Europäisches Patentamt
European Patent Office
Office européen des brevets

(19)

(11) Publication number: **0 095 450**
**A2**

(12) **EUROPEAN PATENT APPLICATION**

(21) Application number: 83850126.0

(22) Date of filing: 13.05.83

(51) Int. Cl.[3]: **C 07 D 211/90**
A 61 K 31/445

(30) Priority: 21.05.82 SE 8203177

(43) Date of publication of application:
30.11.83 Bulletin 83/48

(84) Designated Contracting States:
AT BE CH DE FR GB IT LI LU NL SE

(71) Applicant: **Aktiebolaget Hässle**
**Kärragatan 5**
**S-431 83 Mölndal(SE)**

(72) Inventor: **Berntsson, Peder Bernhard**
**Flugsnapparegatan 17A**
**S-431 33 Mölndal(SE)**

(72) Inventor: **Carlsson, Stig Ake Ingemar**
**Vallmovägen 3**
**S-435 00 Mölnlycke(SE)**

(74) Representative: **Wurm, Bengt Runio et al,**
**Patent and Trade Mark Department AB ASTRA**
**S-151 85 Södertälje(SE)**

(54) **Processes for preparing therapeutically active dihydropyridines and intermediates for the processes.**

(57) New compounds useful as therapeutical agents and useful as intermediates for the preparation of other therapeutically active di-hydropyridines, which new compounds are of the formula

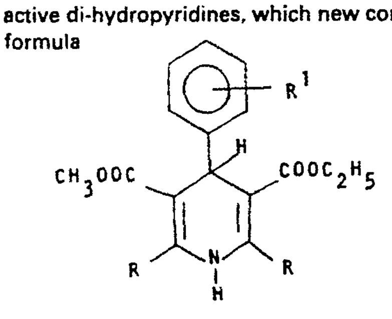

I

wherein $R^1$ in one or two (the same or different) substituents on the phenyl ring selected from the group consisting of $NO_2$, Cl and lower alkyl; R is $-CH_3$, $-CHO$ or

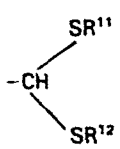

wherein $R^{11}$ and $R^{12}$ are same or different, straight or optionally branched lower alkyl or both together are part of 5-7 membered ring, provided that when one of R is $-CH_3$ the other is $-CHO$ or

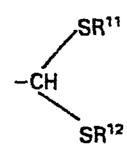

wherein $R^{11}$ and $R^{12}$ have the meaning given above; processes for the preparation of said compounds; pharmaceutical preparations containing said compounds, a method for their therapeutical use and new processes using said compounds as intermediates for the preparation of therapeutically active dihydropyridines.

EP 0 095 450 A2

# Processes for preparing therapeutically active dihydropyridines and intermediates for the processes

## Description

### Field of the invention

The present invention relates to new compounds having valuable therapeutical properties, processes for their preparation, their use as intermediates for preparing other dihydropyridines, new methods for preparing such other dihydropyridines, a method for the treatment of cardiovascular diseases and other disorders where smooth muscle relaxation is therapeutically important in mammals including man and pharmaceutical preparations containing said compounds.

An object of the present invention is to provide new agents for the treatment of cardiovascular diseases and other disorders where smooth muscle relaxation is therapeutically important, particularly agents having antihypertensive properties.

An object of the present invention is also to provide compounds useful as intermediates for the preparation of other dihydropyridines.

A further object of the invention is to provide methods for the preparation of therapeutically active dihydropyridines.

### Prior Art

Compounds of the formula

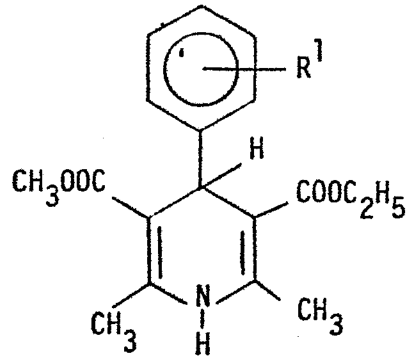

II

wherein $R^1$ is one or two (the same or different) substituents on the phenyl ring selected from the group consisting of $NO_2$, Cl, and lower alkyl are known to possess therapeutic activity (antihypertensive effect). One compound included in the above formula is felodipine, with the formula.

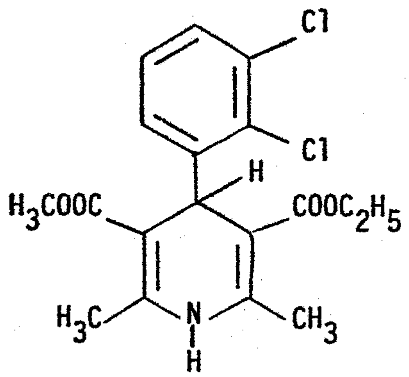

disclosed in US patent No. 4 264 611.

The preparation of the compounds of the formula II has certain problems. The generally employed processes for preparing a particular compound of the formula II above results in the obtainment of a mixture of compounds with regard to the substituents on the ester groups. This mixture of symmetrical and slightly unsymmetrical substances is very difficult to separate into its various components, thus resulting in a highly unpure preparation.

Disclosure of the invention

According to the present invention the above problem is solved by preparing an apparently unsymmetrical intermediate of the formula I below, which can easily be separated from undesired by-products and thereafter transferring the pure substance of formula I by a process, which does not give rise to any by-products, into the end product of formula II. The new process results in end products of a very high degree of purity.

The intermediates of the invention have the formula

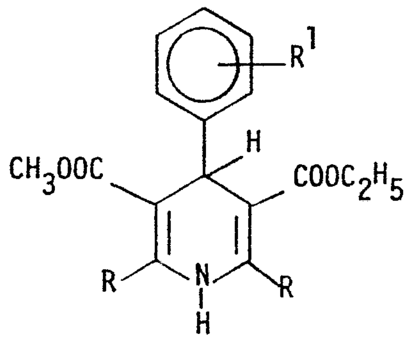

I

wherein $R^1$ is one or two (the same or different) substituents on the phenyl ring selected from the group consisting of $NO_2$, Cl and lower alkyl; R is $-CH_3$, -CHO or 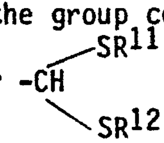 , wherein $R^{11}$ and $R^{12}$ are same or different, straight or optionally branched lower alkyl or both together are part of a 5-7 membered ring, provided that when one of R is $-CH_3$ the other is -CHO or

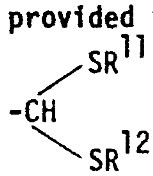 wherein $R^{11}$ and $R^{12}$ have the meaning given above.

Lower alkyl is an alkyl group with 1 - 5 carbon atoms, e.g. methyl, ethyl, propyl, i-propyl, butyl, sec-butyl, i-butyl, tert-butyl.

$R^{11}$ and $R^{12}$ as part of a 5-7 membered ring is in this application defined as

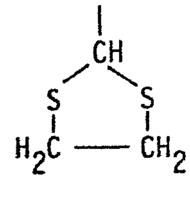
;
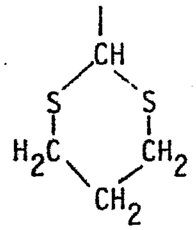
;
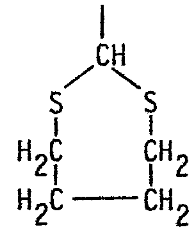

There are two processes according to the invention to transfer the intermediates of the formula I to a therapeutically active compound of the formula II:

A. The process is characterized in that a compound of the formula

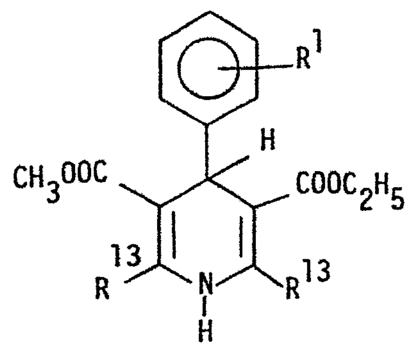
I a

wherein $R^1$ has the meaning given above and $R^{13}$ is $CH_3$ or -CHO, provided that $R^{13}$ in at least one of the two positions is -CHO, is reacted with a compound of the formula

$$H_2NNH-X$$

wherein X is hydrogen or $-CONH_2$, in a first step and with alkali selected from Na-alkoxides, K-hydroxides and Na-hydroxides, in a second step to the formation of a compound of the formula II.

Alternatively the process can also be performed in one single step.

The process is suitably performed at a temperature of 180 - 200 C in highboiling solvents, such as ethylene glycols, octylalcohol, benzylalcohol or triethanolamine.

B. The process is characterized in that a compound of the formula

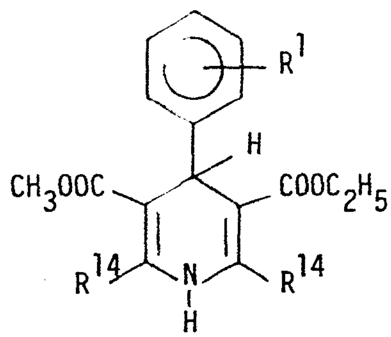

I b

wherein $R^1$ has the meaning given above and $R^{14}$ is $CH_3$ or

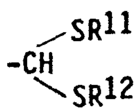

provided that $R^{14}$ in at least one of the two positions is

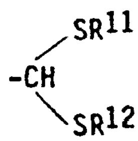

, wherein $R^{11}$ and $R^{12}$ have the meanings given above, is reacted with Raney nickel in a solvent to the formation of a compound of the formula II.

Suitable solvents are for instance methanol, ethanol, dioxane, tetrahydrofurane, benzene, toluene, acetone, methylethylketone, ethylacetate.

Also other catalysts besides Raney nickel can be used, for instance Raney cobalt.

The process is suitably performed at a temperature of 15 - 100°C.

A preferred embodiment of the invention is the preparation according to any of processes A or B of 2,6-dimethyl-4-(2,3-dichlorophenyl)-1,4-dihydropyridine-3,5-dicarboxylic acid-3-methyl-5-ethyl ester.

Other preferred embodiments of the invention are the intermediates:

1. 2-formyl-6-methyl-4-(2,3-dichlorophenyl)-1,4-dihydropyridine-3,5-dicarboxylic acid-3-methyl-5-ethyl ester.

2. 6-formyl-2-methyl-4-(2,3-dichlorophenyl)-1,4-dihydropyridine-3,5-dicarboxylic acid-3-methyl-5-ethyl ester.

3. 2-(1,3-dithian-2-yl)-6-methyl-4-(2,3-dichlorophenyl)-1,4-dihydropyridine-3,5-dicarboxylic acid-3-methyl-5-ethyl ester.

4. 2-bis(propylthio)methyl-6-methyl-4-(2,3-dichlorophenyl)-1,4-dihydropyridine-3,5-dicarboxylic acid-3-methyl-5-ethyl ester.

The compounds of formula I are - besides being useful as intermediates - also effective as antihypertensive agents.

The substances are intended to be administered orally or parenterally for acute and chronic treatment of above mentioned cardiovascular disorders.

The new compounds of formula I are obtained according to methods known _per se_.

Thus,

a$^1$) a compound of the formula

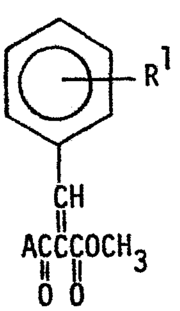

III a

is reacted with a compound of the formula

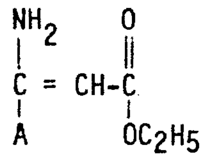

IV a

wherein $R^1$ has the meaning given above and A is $-CH_3$ or

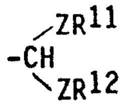

, wherein Z is oxygen or sulphur and $R^{11}$ and $R^{12}$ have the meanings given above, provided that A in at least one of the two positions is

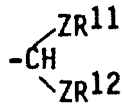

wherein Z, $R^{11}$ and $R^{12}$ have the meanings given above, in a first step and, in the case of Z is oxygen, with HCl in second step to the formation of a compound of the formula I; or

a²) a compound of the formula

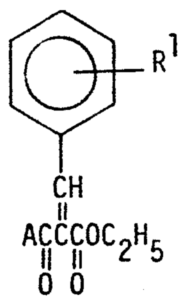

III b

is reacted with a compound of the formula

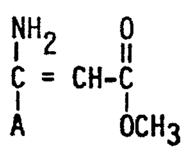

IV b

wherein $R^1$ and A have the meanings given above, in a first step and, in the case of Z is oxygen, with HCl in a second step to

the formation of a compound of the formula I; or

$b^1$) a compound of the formula

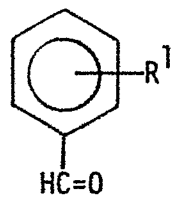

V

is reacted with compounds of the formulas

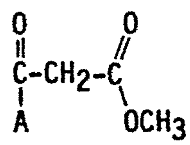

VI a

and

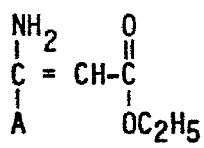

IV a

wherein $R^1$ and A have the meanings given above, in a first step and, in the case of Z is oxygen, with HCl in a second step to the formation of a compound of the formula I; or

$b^2$) a compound of formula V is reacted with compounds of the formulas

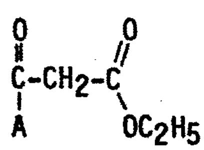

VI b

and

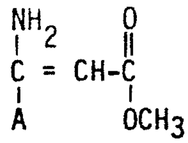

IV b

wherein $R^1$ and A have the meanings given above, in a first step and, in the case of Z is oxygen, with HCl in a second step to the formation of a compound of the formula I; or

$c^1$) a compound of formula III a above is reacted with a compound of the formula

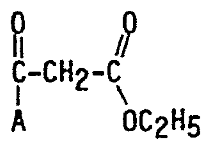

VII a

wherein $R^1$ and A have the meanings given above, in the presence of ammonia in a first step and, in the case of Z is oxygen, with HCl in a second step to the formation of a compound of the formula I; or

$c^2$) a compound of formula III b above is reacted with a compound of the formula

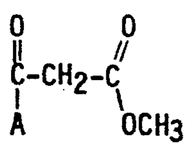

VII b

wherein $R^1$ and A have the meanings given above, in the presence of ammonia in a first step and, in the case of Z is oxygen, with HCl in a second step to the formation of a compound of the formula I; or

d) a compound of formula V above, is reacted with the compounds of formulas VI a and VI b, wherein $R^1$ and A have the meanings given above, in the presence of ammonia in a first step and, in the case of Z is oxygen, with HCl in a second step to the formation of a compound of the formula I; or

e) a compound of the formula

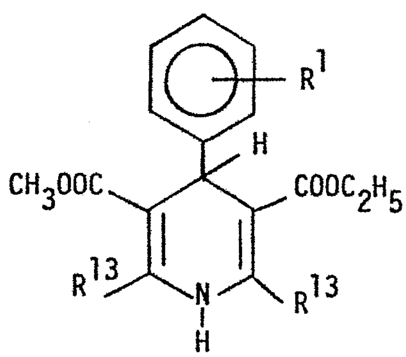

I a

wherein $R^1$ has the meaning given above and $R^{13}$ is $CH_3$ or -CHO, provided that $R^{13}$ in at least one of the two positions is -CHO, is reacted with straight or optionally branched lower alkyl or aryl mercaptanes or with $\alpha,\omega$-dimercapto alkanes to the formation of a compound of the formula

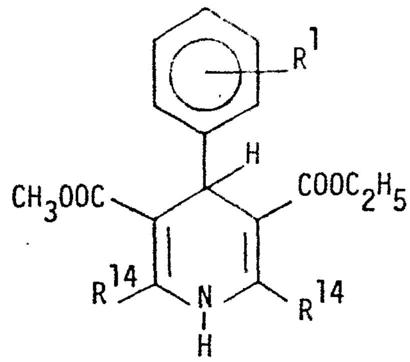

I b

wherein $R^1$ has the meaning given above and $R^{14}$ is $CH_3$ or

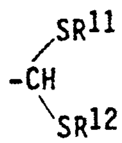

,wherein $R^{11}$ and $R^{12}$ have the meanings given above, provided that $R^{14}$ in at least one of the two positions is

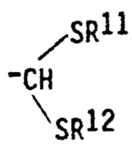

; or

f) a compound of the formula

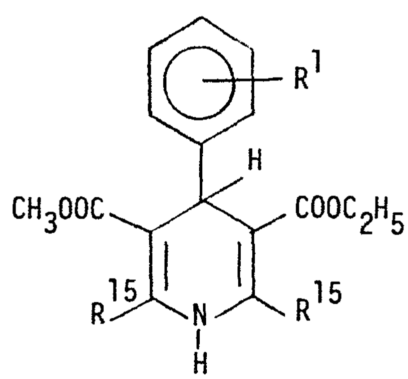

VIII

wherein $R^1$ has the meaning given above and $R^{15}$ is $-CH_3$ or

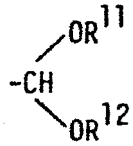 , wherein $R^{11}$ and $R^{12}$ have the meanings given above, provided that $R^{15}$ in at least one of the two positions is 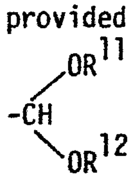 , is reacted with straight or optionally branched lower alkyl or aryl mercaptanes or with $\alpha,\omega$-dimercapto alkanes to the formation of a compound of the formula I b, wherein $R^1$ and $R^{14}$ have the meanings given above.

The reactions $a^1$ - f) are carried out in the presence or absence of a solvent, preferably in the presence of water or an organic solvent. The reactions are carried out at a temperature of 20-200°C.

The invention also relates to any embodiment of the process of which one starts from any compound obtained as an intermediate in any process step and one carries out the lacking process step, or one breaks off the process at any step, or at which one forms a starting material under the reaction conditions, or at which a reaction component possibly in the form of its salt is present.

The starting materials are known or may, if they are novel, be obtained according to processes known per se.

In clinical use the compounds of the invention are usually administered orally, or rectally in the form of a pharmaceutical preparation, which contains the active component as free base in combination with a pharmaceutically acceptable carrier. The carrier may be a solid, semisolid or liquid diluent or a capsule. These pharmaceutical preparations are a further object of the invention.

Usually the amount of active compound is between 0.1 and 99 % by weight of the preparation, suitably between 0.5 and 20 % by weight in preparations for injection and between 2 and 50 % by weight in

preparations for oral administration.

In the preparation of pharmaceutical preparations containing a compound of the present invention in the form of dosage units for oral administration the compound elected may be mixed with a solid, pulverulent carrier, as e.g., with lactose, saccharose, sorbitol, mannitol, starch, such as potato starch, corn starch, amylopectin, cellulose derivatives or gelatine, as well as with an antifriction agent such as magnesium stearate, calcium steareate, polyethyleneglycol waxes or the like, and be pressed into tablets. If coated tablets are wanted, the above prepared core may be coated with concentrated solution of sugar, which solution may contain, e.g., gum arabicum, gelatine, talc, titandioxide or the like. Furthermore, the tablets may be coated with a lacquer dissolved in an easily volatile organic solvent or mixture of solvents. To this coating a dye may be added in order to easily distinguish between tablets with different active compounds or with different amounts of the active compound present.

In the preparation of soft gelatine capsules (pearl-shaped, closed capsules), which consist of gelatine and, e.g., glycerine, or in the preparation of similar closed capsules, the active compound is mixed with a vegetabile oil. Hard gelatine capsules may contain granules of the active compound in combination with a solid, pulverulent carrier as lactose, saccharose, sorbitol, mannitol, starch (as, e.g., potato starch, corn starch or amylopectin), cellulose derivatives or gelatine.

Dosage units for rectal administration bay be prepared in the form of suppositories, which contain the active substance in a mixture with a neutral fat base, or they may be prepared in the form of gelatine-rectal capsules which contain the active substance in a mixture with a vegetable oil or paraffin oil.

Liquid preparations for oral administration may be present in the form of sirups or suspensions, e.g. solutions containing from about 0.2 % by weight to about 20 % by weight of the active substance described, glycerol and propylene glycol. If desired, such liquid preparations may contain colouring agents, flavouring agents, saccharine and carboxymethylcellulose as a thickening agent.

The daily dose of the active substance varies and is dependent on the type of administration, but as a general rule it is 10 to 1000 mg/day of active substance at peroral administration.

Best mode of carrying out the invention

The following illustrates the principles and the adaption of the invention, however, without limiting it thereto.

Example 1 (method $a^1$)

Preparation of 2-formyl-6-methyl-4-(2,3-dichlorophenyl)-1,4-di--hydropyridine-3,5-dicarboxylic acid-3-methyl-5-ethyl ester

A mixture of 43,0 g of 2,3-dichlorobenzylidene-dimethoxyacetyl--acetic acid-methyl ester and 16,7 g of 3-aminocrotonic acid-ethyl ester in 200 ml of pyridine was refluxed for 4 hours. The mixture was then evaporated and the residue dissolved in 300 ml of methylene chloride. The resulting solution was washed twice with water. Drying and evaporation of the organic phase gave a residue, which was further purified by dissolving in 1500 ml of hot petroleum ether (60 - 80$^o$), treatment with charcoal followed by hot filtration and evaporation. This gave 46,4 g of 2-dimethoxymethyl--6-methyl-4-(2,3-dichlorophenyl)-1,4-dihydropyridine-3,5-dicarbox--ylic acid-3-methyl-5-ethyl ester as a yellow oil.

5 g of 2-dimethoxymethyl-4-(2,3-dichlorophenyl)-6-methyl-1,4-di--hydropyridine-3,5-dicarboxylic acid-3-methyl-5-ethyl ester was dissolved in 60 ml of acetone, to which was added 6 ml of 6N HCl. The reaction mixture was left at ambient temperature for 3 hours and was then neutralized with a solution of $NaHCO_3$. After evaporation of the acetone the mixture was filtered and washed with water. Thus was obtained 3,8 g of the title compound. Mp. 155 C.

In a similar way as the first part above 2-(1,3-dithian-2-yl)-6--methyl-4-(2,3-dichlorophenyl)-1,4-dihydropyridine-3,5-dicarboxy--lic acid-3-methyl-5-ethyl ester (Mp. 158°C) was prepared.

Example 2 (method $a^2$)

Preparation of 6-formyl-2-methyl-4-(2,3-dichlorophenyl)-1,4-di--hydropyridine-3,5-dicarboxylic acid-3-methyl-5-ethyl ester

41,2 g of 2,3-dichlorobenzylidene-diethoxyacetyl-acetic acid-ethyl ester and 12,6 g of 3-aminocrotonic acid-methyl ester were dissolved in 75 ml of tert. butanol. The reaction mixture was left at ambient temperature for 7 days and was then evaporated. This gave 38,0 g of 6-diethoxymethyl-2-methyl-4-(2,3-dichlorophenyl)-1,4-dihydropyridine-3,5-dicarboxylic acid-3-methyl-5-ethyl ester as an oil.

9.0 g of 6-diethoxymethyl-2-methyl-4-(2,3-dichlorophenyl)-1,4-dihydropyridine-3,5-dicarboxylic acid-3-methyl-5-ethyl ester was dissolved in 40 ml of acetone, to which was added 8,5 ml of 6N HCl. The mixture was left at ambient temperature for 4 hours and was then neutralized with a solution of $NaHCO_3$. After evaporation the residue was filtered and washed with water. Thus was obtained the title compound. Mp. 130°C.

Example 3 (method f)

Preparation of 2-bis(propylthio)methyl-6-methyl-4-(2,3-dichloro--phenyl)-1,4-dihydropyridine-3,5-dicarboxylic acid-3-methyl-5--ethyl ester

A mixture of 6,0 g of 2-dimethoxymethyl-6-methyl-4-(2,3-dichloro--phenyl)-1,4-dihydropyridine-3,5-dicarboxylic acid-3-methyl-5--ethyl ester, 30 ml of propylmercaptan and 2 drops of concentrated sulphuric acid was refluxed for 48 hours. After evaporation the residue was dissolved in methylene chloride and washed twice with 2N NaOH and once with water. Drying and evaporation gave 3,3 g of the title compound. Mp. 115°C.

Example 4 (process A)

Preparation of 2,6-dimethyl-4-(2,3-dichlorophenyl)-1,4-dihydro--pyridine-3,5-dicarboxylic acid-3-methyl-5-ethyl ester

A mixture of 3,8 g of 2-formyl-4-(2,3-dichlorophenyl)-6-methyl-1,4--dihydropyridine-3,5-dicarboxylic acid-3-methyl-5-ethyl ester, 1,9 g of potassium hydroxide and 1,4 g of 85 % hydrazine hydrate in 20 ml of diethylene glycol is refluxed for one hour, the water formed is removed and the temperature of the solution is allowed to rise to 180°C when refluxing is continued for four hours more. The cooled solution is diluted with 50 ml of water and extracted with methylene chloride. After evaporation of methylene chloride the residue was recrystallized from isopropylether. Thus was obtained the title compound in 78 % yield. Mp. 144°C.

Example 5 (process B)

Preparation of 2,6-dimethyl-4-(2,3-dichlorophenyl)-1,4-dihydro--pyridine-3,5-dicarboxylic acid-3-methyl-5-ethyl ester

2,00 g of 2-bis(propylthio)methyl-6-methyl-4-(2,3-dichlorophenyl)--1,4-dihydropyridine-3,5-dicarboxylic acid-3-methyl-5-ethyl ester was dissolved in 75 ml of ethanol. Raney nickel was added and the mixture was stirred at room temperature for 18 hours. Filtration, evaporation and recrystallization from isopropylether gave the title compound in 65 % yield. Mp. 144°C.

For the compounds prepared according to examples 1-5 $^{1}$H-NMR and $^{13}$C-NMR have been obtained.

Example 6

The following examples illustrate the preparation of pharmaceutical compositions of the invention. The wording "active substance" denotes a compound according to the present invention or a salt thereof and preferably the compound according to Example 1.

Formulation A

A syrup containing 2 % (weight per volume) of active substance was prepared from the following ingredients:

| | |
|---|---|
| Active substance | 2.0 g |
| Saccharine | 0.6 g |
| Sugar | 30.0 g |
| Glycerine | 5.0 g |
| Flavouring agent | 0.1 g |
| Ethanol 96 % | 10.0 g |
| Distilled water | ad 100.0 g |

Sugar, saccharine and the active substance were dissolved in 60 g of warm water. After cooling, glycerine and solution of flavouring agents dissolved in ethanol were added. To the mixture water was then added to 100 ml.

Formulation B

Active substance (250 g) was mixed with lactose (175.8 g), potato starch (169.7 g) and colloidal silicic acid (32 g). The mixture was moistened with a 10 % of gelatine and was granulated through a 12 mesh sieve. After drying potato starch (160 g), talc (50 g) and magnesium stearate (5 g) were admixed and the mixture thus obtained was pressed into tablets (10.000), each containing 25 mg of active substance. The tablets are provided with a breaking score to give another dose than 25 mg or to give multiples thereof when broken.

Formulation C

Granules were prepared from active substance (250 g), lactose (175.9 g) and an alcoholic solution of polyvinylpyrrolidone (25 g). After the drying step the granules were mixed with talc (25 g), potato starch (40 g) and magnesium stearate (2.50 g) and were pressed into 10.000 tablets being biconvex. These tablets are coated with a 10 % alcoholic solution of shellac and thereupon with an aqueous solution containing saccharose (45 %), gum arabicum (5 %), gelatine (4 %) and dyestuff (0.2 %). After the first five coatings talc and powdered sugar were used for powdering. The priming coat was then coated with a 66 % sugar syrup and polished with a 10 % carnauba wax solution in carbon tetrachloride.

CLAIMS

1. A compound of the formula

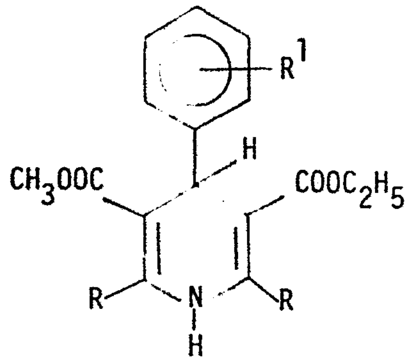

I

wherein $R^1$ in one or two (the same or different) substituents on the phenyl ring selected from the group consisting of $NO_2$, Cl and lower alkyl; R is $-CH_3$, -CHO or 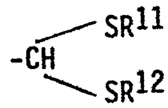 , wherein $R^{11}$ and $R^{12}$ are same or different, straight or optionally branched lower alkyl or both together are part of a 5-7 membered ring, provided that when one of R is $-CH_3$ the other is -CHO or 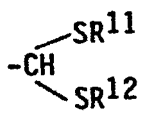 , wherein $R^{11}$ and $R^{12}$ have the meaning given above.

2. A compound according to claim 1;

1) 2-formyl-6-methyl-4-(2,3-dichlorophenyl)-1,4-dihydropyri--dine-3,5-dicarboxylic acid-3-methyl-5-ethyl ester;

2) 6-formyl-2-methyl-4-(2,3-dichlorophenyl)-1,4-dihydropyri--dine-3,5-dicarboxylic acid-3-methyl-5-ethyl ester;

3) 2-(1,3-dithian-2-yl)-6-methyl-4-(2,3-dichlorophenyl)-1,4--dihydropyridine-3,5-dicarboxylic acid-3-methyl-5-ethyl ester;

4) 2-bis(propylthio)methyl-6-methyl-4-(2,3-dichlorophenyl)--1,4-dihydropyridine-3,5-dicarboxylic acid-3-methyl-5-ethyl ester.

3. A process for preparing a compound of the formula

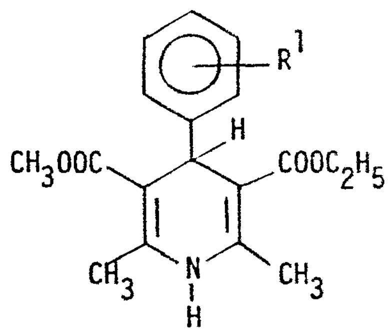

II

wherein $R^1$ is one or two (the same or different) substituents on the phenyl ring selected from the group consisting of $NO_2$, Cl and lower alkyl characterized in that a compound of the formula

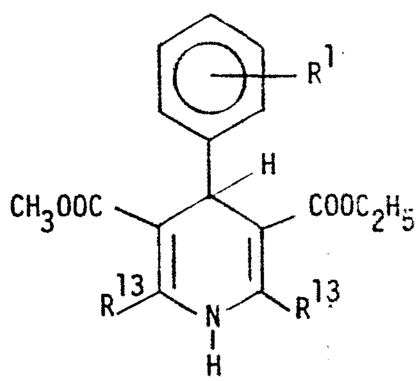

I a

wherein $R^1$ has the meaning given above and $R^{13}$ is $CH_3$ or -CHO provided that $R^{13}$ in at least one of the two positions is -CHO, is reacted with a compound of the formula

$H_2NNH-X$

wherein X is hydrogen or $-CONH_2$, in a first step and with alkali, in a second step to the formation of a compound of the formula II.

4. A process according to claim 3 characterized in that 2,6-dimethyl-4-(2,3-dichlorophenyl)-1,4-dihydropyridine-3,5-dicarboxylic acid-3-methyl-5-ethyl ester is prepared.

5. A process for preparing a compound of the formula

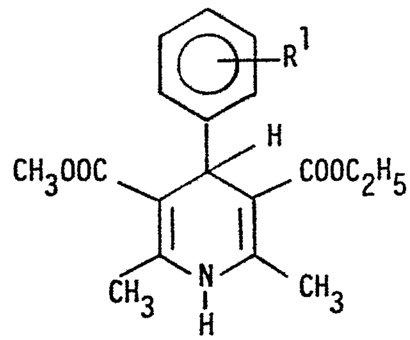

II

wherein $R^1$ is one or two (the same or different) substituents on the phenyl ring selected from the group consisting of $NO_2$, Cl and lower alkyl characterized in that a compound of the formula

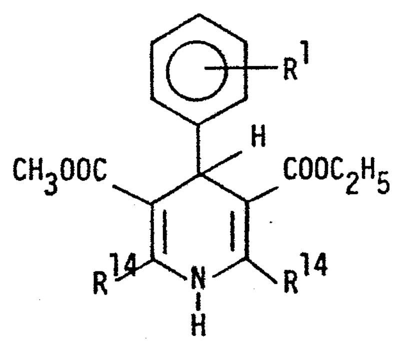

I b

wherein $R^1$ has the meaning given above and $R^{14}$ is $CH_3$ or

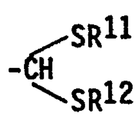

provided that $R^{14}$ in at least one of the two is

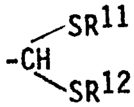 , wherein $R^{11}$ and $R^{12}$ have the meanings given above, is reacted with a Raney catalyst in a solvent to the formation of a compound of the formula II.

6. A process according to claim 5 characterized in that 2,6-dimethyl-4-(2,3-dichlorophenyl)-1,4-dihydropyridine-3,5-dicarboxylic acid-3-methyl-5-ethyl ester is prepared.

7. A process for preparing a compound of the formula

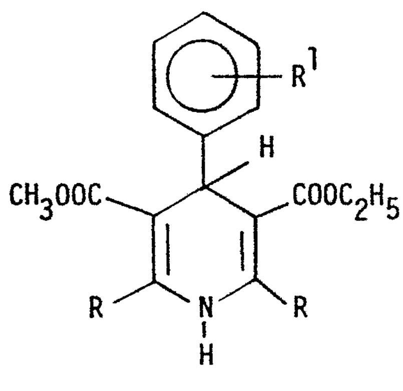

I

wherein $R^1$ in one or two (the same or different) substituents on the phenyl ring selected from the group consisting of $NO_2$, Cl and lower alkyl; R is $-CH_3$, -CHO or 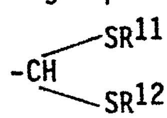 , wherein $R^{11}$ and $R^{12}$ are same or different, straight or optionally branched lower alkyl or both together are part of a 5-7 membered ring, provided that when one of R is $-CH_3$ the other is -CHO or 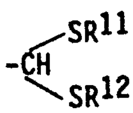 , wherein $R^{11}$ and $R^{12}$ have the meanings given above, characterized in that

$a^1$) a compound of the formula

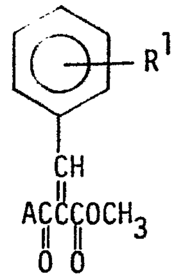

III a

is reacted with a compound of the formula

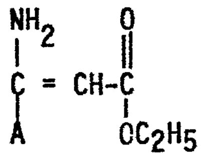

IV a

wherein $R^1$ has the meaning given above and A is $-CH_3$ or

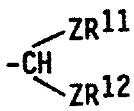

, wherein Z is oxygen or sulphur and $R^{11}$ and $R^{12}$ have the meanings given above, provided that A in at least one on the two positions is

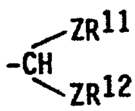

, wherein Z, $R^{11}$ and $R^{12}$ have the meanings given above, in a first step and, in the case of Z is oxygen, with HCl in a second step to the formation of a compound of the formulad I; or

$a^2$) a compound of the formula

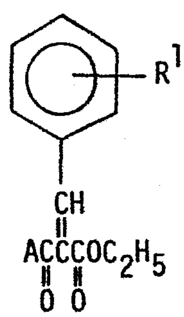

III b

is reacted with a compound of the formula

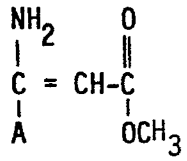

IV b

wherein $R^1$ and A have the meanings given above, in a first step and, in the case of Z is oxygen, with HCl in a second step to the formation of a compound of the formula I; or

$b^1$) a compound of the formula

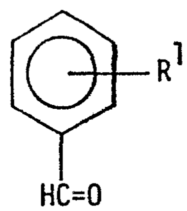

V

is reacted with compounds of the formulas

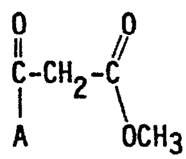

VI a

and

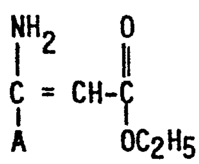

IV a

wherein $R^1$ and A have the meanings given above, in a first step and, in the case of Z is oxygen, with HCl in a second step to the

formation of a compound of the formula I; or

$b^2$) a compound of formula V is reacted with compounds of the formulas

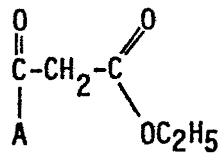

VI b

and

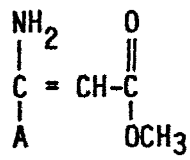

IV b

wherein $R^1$ and A have the meanings given above, in a first step and, in the case of Z is oxygen, with HCl in a second step to the formation of a compound of the formula I; or

$c^1$) a compound of formula III a above is reacted with a compound of the formula

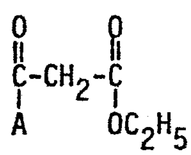

VII a

wherein $R^1$ and A have the meanings given above, in the presence of ammonia in a first step and, in the case of Z is oxygen, with HCl in a second step to the formation of a compound of the formula I; or

c²) a compound of formula V is reacted with compounds of the formulas

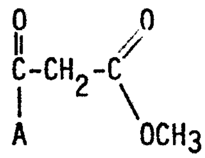

VII a

wherein $R^1$ and A have the meanings given above, in the presence of ammonia in a first step and, in the case of Z is oxygen, with HCl in a second step to the formation of a compound of the formula I; or

d) a compound of formula V above, is reacted with the compounds of formulas VI a and VI b, wherein $R^1$ and A have the meanings given above, in the presence of ammonia in a first step and, in the case of Z is oxygen, with HCl in a second step to the formation of a compound of the formula I; or

e) a compound of the formula

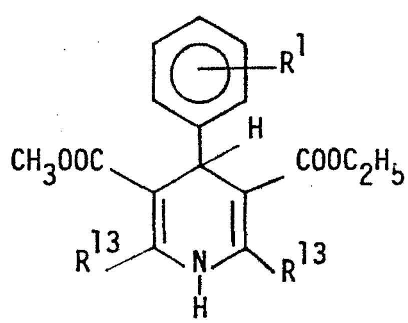

I a

wherein $R^1$ has the meaning given above and $R^{13}$ is $CH_3$ or -CHO, provided that $R^{13}$ in at least one of the two positions is -CHO, is reacted with straight or optionally branched lower alkyl or aryl mercaptanes or with $\alpha,\omega$-dimercapto alkanes to the formation of a compound of the formula

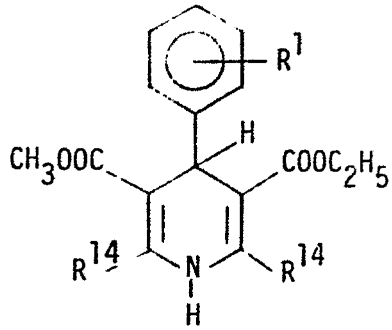

I b

wherein $R^1$ has the meaning given above and $R^{14}$ is $CH_3$ or 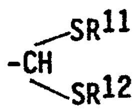 , wherein $R^{11}$ and $R^{12}$ have the meanings given above, provided that $R^{14}$ in at least one of the two positions is 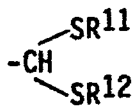 ; or

f) a compound of the formula

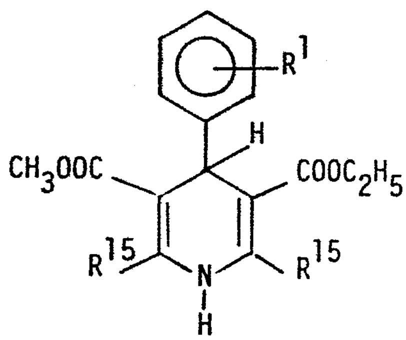

wherein $R^1$ has the meaning given above and $R^{15}$ is $-CH_3$ or 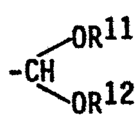 , wherein $R^{11}$ and $R^{12}$ have the meanings given above, provided that $R^{15}$ in at least one of the two positions 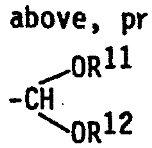 , is reacted with straight or optionally branched lower alkyl or aryl mercaptanes or with $\alpha,\omega$-dimercapto alkanes to the formation of a compound of the formula I b, wherein $R^1$ and $R^{14}$ have the meanings given above.

8. A process according to claim 7 characterized in that a compound according to any of claims 1-2 is prepared.

9. A pharmaceutical preparation comprising as active ingredient a compound according to any of claims 1-2.

10. A pharmaceutical preparation according to claim 9 in dosage unit form.

11. A pharmaceutical preparation according to claims 9-10 comprising the active ingredient in association with a pharmaceutically acceptable carrier.

12. The use of a compound according to any of claims 1-2 for the preparation of a pharmaceutical preparation comprising as an active ingredient an amount of said compound.

13. A method for the treatment of cardiovascular diseases and other disorders where smooth muscle relaxation is therapeutically important, particularly hypertension, in mammals including man, characterized by the administration to a host in need of such treatment of an effective amount of a compound according to any of claims 1-2.

14. A compound according to any of claims 1-3 for use as a drug for the treatment of cardiovascular diseases and other disorders where smooth muscle relaxation is therapeutically important, particularly hypertension.

15. The use of a compound according to any of claims 1-2 as an intermediate for the preparation of therapeutically active dihydro--pyridines.